# EUROPEAN PATENT SPECIFICATION

(11) **EP 1 480 684 B1**
(45) Date of publication and mention of the grant of the patent: **10.12.2008**
(21) Application number: 03743554.2
(22) Date of filing: 03.03.2003
(51) Int. Cl.: A61L 2/04, A23L 3/02, A23L 3/10, B65B 55/10, A23B 4/005, A23C 3/023, A61L 2/26

(54) **METHOD OF HEAT-TREATING PACKAGED PRODUCT**
VERFAHREN ZUR WÄRMEBEHANDLUNG VERPACKTER PRODUKTE
PROCEDE DE TRAITEMENT THERMIQUE D'UN PRODUIT EMBALLE

(30) Priority: 04.03.2002 JP 2002057629
(43) Date of publication of application: 01.12.2004
(73) Proprietor: Kureha Corporation, Tokyo 103-8552 (JP)
(72) Inventor: YAMANE, Kazuyuki, Iwaki-shi, Fukushima 974-8686 (JP); KAWAKAMI, Yukichika, Iwaki-shi, Fukushima 974-8686 (JP); WAKAMATSU, Akiko, Iwaki-shi, Fukushima 974-8686 (JP); YASUDA, Matsuo, Niihari-gun, Ibaraki 311-3436 (JP); TANAKA, Mikio, Niihari-gun, Ibaraki 311-3436 (JP)
(74) Representative: Barendregt, Frank
(86) International application number: PCT/JP2003/002431
(87) International publication number: WO 2003/074092

(56) References cited:
- EP-A- 1 176 002
- US-A- 4 355 721
- US-A- 4 818 592
- US-A- 6 022 913

## Description

### [TECHNICAL FIELD]

The present invention relates to a method of heat-treating a packaged product (formed by packaging a content material, such as food, beverages, sanitary products or medical materials, with a plastic packaging material or container for packaging or containing the content material) with hot water for sterilization, cooking, etc. More particularly, the present invention relates to a method of heat-treating a product packaged with a plastic material or container (hereinafter inclusively referred to as a "packaging material") in hot water while suppressing opalescence (or whitening) or lowering in gas-barrier property of the plastic packaging material, and also a packaged product thus heat-treated.

### [BACKGROUND ART]

Hitherto, plastic packaging materials have been widely used for packaging or as containers for food or beverage, sanitary products and medical materials. Among such plastic packaging materials, some materials have been known as causing opalescence or a lowering in transparency after a heat treatment when such a packaging material is heat-treated by itself or in a form of a packaged product enclosing, e.g., food or a medical material for the purpose of sterilization and/or cooking at a temperature on the order of 60-140°C within tap water or industrial water. (Industrially, a heat-treatment using hot water at 60- 100°C is frequently called a boiling treatment, and one using hot water above 100°C is frequently called a retort treatment.) The opalescence of the packaging material causes problems, such as limited observation of the content material state, and poor appearance, and is sometimes accompanied with a lowering in gas-barrier property. The lowering in gas-barrier property of the packaging material results in problems, such as shortage of shelf-life of the packaged product. There has not yet been developed an effective method for alleviating the above-mentioned problems accompanying the heat-treatment of packaged products in hot water.

### [DISCLOSURE OF INVENTION]

An object of the present invention is to provide a method of heat-treating a packaged product with a plastic material with hot water while suppressing the opalescence of the packaging material causing a poor appearance and a limited clarity and further leading to a lowering in gas-barrier property.

The present invention, provides a heat-treating method according to claim 1. Some details of history of our study with the above objects through which we have arrived at the present invention will be referred to below.

We have studied the opalescence due to hot-water treatment of a plastic packaging material causing disadvantage in appearance and transparency, and recognized that the opalescence is attributable to at least a hydrophilic resin layer contained in the packaging material. More specifically, if a hydrophilic resin layer is present in the packaging material to be treated with hot water, hot water molecules are caused to contact the hydrophilic resin layer not only in the case where the hydrophilic resin layer constitutes a surface layer of the packaging material directly contacting the hot water, as a matter of course, but also in the case where it constitutes an inner layer which does not directly contact the hot water due to coverage with a hydrophobic resin layer (including an inner surface layer not contacting the hot water in addition to an intermediate layer between two surface layers), due to diffusion of hot water molecules through microscopic pores in or through gaps between hydrophobic polymer molecules constituting the surface layer, thereby reaching the hydrophilic resin layer to whiten the hydrophilic resin layer by forming a cluster of water molecules attached to the hydrophilic resin layer, according to our understanding. In this instance, if the hydrophilic resin is also hydrolyzable, the opalescence is promoted in association with a molecular weight-decreasing effect due to the hydrolyzation, and if the hydrophilic resin layer forms a surface layer, the molecules causing the surface layer is partially dissolved to cause a surface roughening which also promotes the opalescence. These opalescence-promoting phenomena are further enhanced by an increase in hot water temperature reflecting an increase in kinetic energy of water molecules and therefore are more noticeably caused in the retort treatment using hot water at a temperature exceeding 100°C than in the boiling treatment using hot water temperature at a temperature of at most 100°C.

However, the above-mentioned opalescence during hot-water treatment of a packaging material including a hydrophilic resin layer has been recognized as an inevitable phenomenon accompanying the use of a hydrophilic resin. Accordingly, it has been an actual practice to obviate the use of a packaging material including a hydrophilic resin layer for a packaged product subjected to a boiling or retort hot water treatment in spite of the fact that many hydrophilic resin layers are known to have excellent properties, inclusive of gas-barrier property.

In contrast thereto, we had an idea that if the percentage of free water molecules is lowered to decrease the kinetic energy of the water molecules by adding a water-soluble compound in hot water to cause hydration thereof with the water molecules, the above-mentioned opalescence of a packaging material including a hydrophilic resin layer might be suppressed. Based on the concept, we have experimentally confirmed that the addition of a water-soluble compound in the hot-water treatment exhibits a remarkable opalescence-suppression effect, thereby arriving at the present invention.

### [DETAILED DESCRIPTION OF THE INVENTION]

The plastic packaging material used in the present invention may comprise either a single-layered film or container comprising one hydrophilic resin layer, or a multi-layered film or container comprising at least two resin layers including at least one hydrophilic resin layer.

The film-form packaging material may comprise, e.g., a non-stretched film, a stretched film, a heat-shrinkable film, or a non-heat-shrinkable film.

Such a film may, for example, be produced by melt-extrusion of a plastic material through a flat die.

A non-stretched film may be produced by melt-extrusion, followed by shaping into a final film form at a relatively high temperature in the course of cooling. A stretched film or a heat-shrinkable film may, for example, be produced through a process wherein a sheet formed by melt-extrusion is stretched while being cooled, or is cooled, re-heated and stretched, optionally followed by heat-setting. For the film formation, a sheet may be melt-extruded through a flat die and then stretched uniaxially, successively biaxially or simultaneously biaxially by the roller process, the tenter process or a combination of these. Alternatively, it is also possible to effect biaxial stretching by the inflation process using a circular die. The film after the stretching may be subjected to heat-shrinkability adjustment for providing a heat shrinkability of zero or a desired degree by an appropriate annealing treatment under no tension or application of an appropriate degree of tension.

As described above, the film may comprise a single layer of hydrophilic resin or may be formed into a multi-layer structure together with another resin layer. The multilayer formation may be effected by lamination, coating or co-extrusion.

The lamination may include: wet lamination, dry lamination, extrusion lamination, hot melt lamination, non-solvent lamination, etc., which may be selectively used as desired. The coating may include: provision of a moisture-proof coating or a moisture-proof lamination layer which may be selectively used as desired.

A flat film or a film formed by slitting of an inflation film of a large lay-flat width may be formed into a bag, a pouched., filled with a content material, such as foods, sanitary products or medical materials and hermetically sealed, before being subjected to hot water treatment according to the present invention.

The filling and packaging of a content material may also be effected by using an automatic packaging machine, by which a film is formed into a tube by center seaming while filling the tube with a content material, such as food, to package the content material.

Examples of the packaging material in the form of a container may include: trays or cups formed by subjecting a plastic sheet of a single layer or multi-layers to sheet-forming technique, such as vacuum forming or pressure forming, and bottles formed by blow molding.

Similarly as the films, these containers may be subjected to the hot water treatment according to the present invention after being filled with a content material.

The hydrophilic resin giving the hydrophilic resin layer of the packaging material may include: polyamides; polyesters; polyorganic acids, such as polyamino acids; vinyl alcohol (co-)polymers, and acid-modified olefin (co-)polymers, and the degree of hydrophilicity suitable to be treated according to the method of the present invention may be judged based on the intended effect depending on whether or not the film is opacified when subjected to an ordinary hot water treatment. More specifically, when an, e.g., 50 µm-thick single-layered film of a hydrophilic resin is subjected to a test of immersion in still hot water at 100°C for 30 min., if the film is hydrolyzed ( the hydrophilic resin layer should be disposed as an inner layer in this case) or exhibits a haze value of 20% or higher (that is the degree of opalescence to be prevented according to the present invention), a packaging material including the hydrophilic resin layer may be a suitable object to be treated by the hot water treatment method according to the present invention. According to this standard, aromatic polyesters do not generally come under hydrophilic resins, but aliphatic polyesters generally come under hydrophilic resins. Incidentally, it is also possible to use a mixture of two or more species of hydrophilic resins, or a mixture of at least one species of hydrophobic resin and at least one species of hydrophilic resin. Also, in this case, the required degree of hydrophilicity can be judged according to the above-mentioned hot water immersion test.

For providing a packaging material of multi-layer structure, the above-mentioned hydrophilic resin layer may be laminated with a surface layer (outer surface layer) which may comprise a hydrophilic resin free from hydrolyzability selected from the above-mentioned class of hydrophilic resins or also a hydrophobic resin. Preferred examples thereof may include: polyolefins or olefin copolymers, such as polyethylene and polypropylene; aromatic polyesters, such as polyethylene terephthalate, polybutylene terephthalate and polyethylene naphthalate; (aliphatic or aromatic) polyamides; and styrene resins comprising homopolymer and copolymers of styrene. These surface layer resins may be used singly or in mixture of two or more species, and can also be suitably used as a resin constituting an inner layer (i.e., an intermediate layer or an inner surface layer).

In a preferred embodiment of the present invention, a gas-barrier resin layer is included as a hydrophilic resin layer. This embodiment is preferred because a heat-treatment method, as by boiling or retorting, is frequently used for sterilizing a packaged product enclosing a content material such as food, and such a content material is preferably enclosed with a packaging material including an (oxygen) gas-barrier resin layer for long-term preservation thereof, whereas many resins showing good gas-barrier property are hydrophilic.

### [Hydrophilic gas-barrier resin layer]

Preferred examples of hydrophilic gas-barrier resin may include: glycolic acid (co-)polymer(PGA), ethylene-vinyl alcohol copolymer (EVOH), polyvinyl alcohol, and polyamide (co-)polymer. These hydrophilic gas-barrier resins are described below in some detail.

### Glycolic acid (co-)polymer (PGA)

A hydrophilic gas-barrier resin particularly preferably used in the present invention is glycolic acid (co-)polymer. The reason is that glycolic acid (co-)polymer exhibits extremely excellent gas-barrier property and moisture-barrier property but its use in a packaging material subjected to boiling or retort sterilization has been restricted because of its high hydrophilicity (or hydrolyzability) causing opalescence during the heat-treatment thereof by boiling or retorting, whereas the problem can be effectively solved by the present invention.

Glycolic acid (co-)polymer preferably used in the present invention is crystalline and has a melting point. Such a glycolic acid (co-)polymer may be produced by polycondensation of glycolic acid or an ester or salt thereof, or by ring-opening polymerization of glycolide which is a bimolecular cyclic ester of glycolic acid as represented by a reaction formula shown below. Particularly, in order to produce a glycolic acid (co-)polymer for providing a formed product, such as a sheet or a film, expected to show a high strength, the latter ring-opening polymerization of glycolide is preferred.

By using glycolide alone, glycolic acid homopolymer can be obtained. By using glycolide and a smaller amount of another comonomer within a extent of retaining the crystallinity, i.e., within an range of retaining a melting point, a glycolic acid copolymer can be obtained. Examples of the comonomer may include: cyclic monomers, inclusive of ethylene oxalate (i.e., 1,4-dioxane-2,3-dione); lactide, and lactones, such as β-propiolactone, β -butyrolactone, pivalolactone, γ-butyrolactone, δ -valerolactone, β-methyl-δ-valerolactone, and ε -caprolactone; trimethylene carbonate, and 1,3-dioxane; hydroxycarboxylic acids, such as lactic acid, 3-hydroxypropanoic acid, 3-hydroxybutanoic acid, 4-hydroxybutanoic acid and hydroxycaproic acid, and their alkyl esters; substantially equal molar mixtures of aliphatic diols, such as ethylene glycol and 1,4-butane diol with aliphatic dicarboxylic acids, such as succinic acid and adipic acid, and their alkyl esters; and combinations of two or more species of such co-monomers. As a starting material for polymerization, it is also possible to use a combination of glycolide and glycolic acid.

Among these comonomers, cyclic monomers, such as lactide, caprolactone and trimethylene carbonate; and hydroxycarboxylic acids, such as lactic acid and glycolic acid, are preferred, because of easy copolymerizability and capability of providing copolymers having excellent physical properties. Such a comonomer is used in a proportion of ordinarily at most 45 wt.%, preferably at most 30 wt.%, more preferably at most 10wt.%. The reason is that a copolymer having lost crystallinity is liable to result in remarkable lowering in properties, such as heat-resistance, gas-barrier property and mechanical strength.

A polymerization apparatus for providing such a crystalline glycolic acid (co-)polymer may appropriately be selected from various apparatus including those of an extruder-type, a vertical type having paddle blades, a vertical type having helical ribbon blades, a laterally disposed extruder or kneader type, an ampoule-type, and a tube-type.

Other preferable hydrophilic gas-barrier resins are described below.

### Ethylene-vinyl alcohol copolymer (EVOH)

Ethylene-vinyl alcohol copolymer (EVOH) used in the present invention may preferably have an ethylene content of 20-60mol% and a saponification degree of at least 95mol%. Such ethylene-vinyl alcohol copolymer is generally sold commercially and readily available.

### Polyamide (PA)

As a polyamide, MXD6 nylon (polymetaxylylene adipamide) is preferred.

In the case of providing a multi-layered packaging material, it is possible to insert an optional intermediate layer between two surface layers. An example thereof is the above-mentioned hydrophilic gas-barrier resin layer (which however can also be a surface layer). In addition thereto, it is also possible to insert one or more layers of various resin material for the purpose of adjusting the strength, improving the inter-layer adhesion, etc. Such an intermediate layer may preferably be formed of an extrudable resin material.

Examples of resins constituting such an adhesive resin layer may include: carboxylated polyolefin, epoxidized polyolefin, ethylene-vinyl acetate copolymer, ionomer, polyurethane, epoxy resin, styrene-butadiene-styrene copolymer (SBS), styrene-ethylene-butadiene-styrene copolymer (SEBS), polychloroprene, styrene-butadiene copolymer rubber (SBR), and natural rubber (NR).

The carboxylated polyolefin is a polyolefin having a carboxyl group introduced thereinto by modifying a polyolefin with an unsaturated acid monomer, such as acrylic acid, methacrylic acid, or maleic anhydride. The introduction of a carboxylic group may be effected either by copolymerization or grafting. The above mentioned unsaturated acid monomer can also be used in combination with a vinyl monomer, such as methacrylate esters, acrylate esters, or vinyl acetate.

The epoxidized polyolefin is a polyolefin having an epoxy group introduced thereinto by modifying a polyolefin with an epoxy group-containing monomer, such as glycidyl methacrylate. The introduction of an epoxy group may be effected either by copolymerization or grafting. The epoxy group-containing monomer can also be used in combination with a vinyl monomer, such as methacrylate esters, acrylate esters, or vinyl acetate.

Among the above-mentioned adhesive resins, carboxylated polyolefin, epoxidized polyolefin and ethylene-vinyl acetate copolymer, are particularly preferred in view of the adhesiveness and processability. A mixture of these adhesive resins can also be used, as desired, within an extent of not adversely affecting the clarity of the resultant packaging material.

Including the respective layers described above, the packaging material forming the packaged product of the present invention may generally have a total thickness of 10-3000 µm, of which the hydrophilic resin layer may preferably occupy 2 to 30%, while different thicknesses may be adopted depending on the form of the packaging material, such as sheets or containers (including bottles). In this instance, in the case of a relatively long container, such as a bottle, the above-mentioned thicknesses are taken at its body part, and in some cases, the hydrophilic resin layer may be present only at the body part and not present at the mouth or/and the bottom. In the case of inserting an adhesive resin layer, its thickness may preferably be selected from the range of ca. 0.5 to 100 µm.

According to the present invention, a packaging product is formed by enclosing a content material in the above-provided packaging material and is brought into contact with hot water containing a water-soluble compound, preferably by immersion, to effect a heat treatment.

The water-soluble compound may be either an inorganic eletrolyte or a water-soluble organic compound.

The inorganic electrolyte can be an acid or an alkali so as to attain an intended effect of preventing the opalescence of the packaging material, but may preferably be a water-soluble inorganic salt from the view point of safe use. Preferred examples thereof may include: sodium chloride, potassium chloride, magnesium chloride, calcium chloride, aluminum chloride, and sodium sulfate. Among these, chlorides inclusive of sodium chloride, potassium chloride and magnesium chloride are preferably used.

As the water-soluble organic compound, organic acids, ethers and ketones, can be used; and in addition thereto, water-soluble alcohols, such as ethyl alcohol, ethylene glycol and polyethylene glycol are suitably used.

Among the above-mentioned water-soluble compounds, sodium chloride is most suitably used in view of the sanitary aspect and water-solubility.

As for the concentration of the water-soluble compound in hot water, a higher concentration tends to exhibit a higher opalescence-prevention effect, but even a concentration of 1 wt.% exhibits a sufficient effect (Examples 2,3 and 5 described later), and a concentration of 0.1 wt.% or higher is expected to exhibit some effect for some hydrophilic resin. In this regard, it should be noted that the effective concentration level is different depending on the species of hydrophilic resin used, and even the concentration of 0.1 wt.% is substantially higher than a salt concentration (ca. 400 ppm(=0.04 wt.%)) or below that is allowed to be present in tap water which has been conventionally used for boil sterilization, etc., and is a level of concentration that cannot be realized unless a salt is intentionally added.

Through the hot-water treatment of the present invention using hot water containing a water-soluble compound, the resultant hot water-treated packaging film can be provided with a suppressed opalescence, as represented by a lowering in haze of preferably at least 10%, more preferably 20% or higher, compared with the case wherein the hot-water treatment is performed by using hot water to which such a water-soluble compound has not been added. The concentration of a specific water-soluble compound to be added may be determined so as to achieve the above-mentioned opalescence-suppression effect.

The hot-water treatment of the present invention can be achieved by using tap water with a water-soluble compound dissolved therein, instead of ordinary tap water, in a hot-water boiled sterilization apparatus or a hot-water retort sterilization apparatus which has been conventionally used.

The hot-water treatment time may be approximately set in the same manner as in the conventional treatment depending on the size of the content material and the level of sterilization intended by the boil sterilization or retort sterilization. The treatment time ranges from 1 min. (surface sterilization of the content material) to ca. 3 hours for a boil sterilization, and from 5 min. to ca. 1 hour for a retort sterilization.

If a packaging material provided with a heat-shrinkability through a stretching treatment is used as the packaging material, the packaging material causes a heat-shrinkage to tightly adhere to the content material during the hot-water treatment, thereby providing a heat-treatment packaging product exhibiting a further improved transparency.

### [Examples]

Hereinbelow, the present invention will be described more specifically based on Examples, Comparative examples and Reference examples.

Component resins used in the following Examples are represented by abbreviations or symbols in Tables appearing hereinafter, and the meanings thereof are described as follows:
- EVOH: ethylene-vinyl alcohol copolymer ("SOANOL E3803B", made by Nippon Gosei Kagaku K.K.)
- Ny6-66: nylon 6-66 copolymer ("AMILAN CM6001XF", made by Toray K.K.)
- Ad.1: Adhesive 1=acid-modified LLDPE (linear low-density polyethylene)("ADMER NF528", made by Mitsui Kagaku K.K.)
- LLDPE: linear low-density polyethylene ("MOATEC VO398CN", made by Idemitsu Siekiyu Kagaku K.K.)
- Ny6: nylon 6 ("AMILAN CM1021FS4", made by Toray K.K.)
- VLDPE: very low-density polyethylene ("MOATEC V0398CN", made by Idemitsu Sekiyu Kagaku K.K.)
- CPP: direct inflation film of propylene-ethylene random copolymer (made by Kureha Kagaku Kogyo K.K.)
- Ad.2: Adhesive 2= urethane-based adhesive for dry lamination.
- HB-NY: Ny6 (5 µm)/MXD6(5 µm)/NY6 (5 µm) co-extruded film ("SUPERNEAL SPR8H", made by Mitsubishi Kagaku Kohjin Packs K.K.)
- EVA: ethylene-vinyl acetate copolymer ("EVAFLEX V-527-4", made by Mitsui Dupont K.K.)
- PP: polypropylene ("NOVATEC FY6C", made by Nippon Polychem K.K.)
- Ad.3: Adhesive 3= malic acid-modified ethylene-vinyl acetate copolymer ("ADMER VF500", made by Mitsui Kagaku K.K.)
- Ad.4: Adhesive 4=maleic acid-modified polypropylene ("ADMER QB550", made by Mitsui Kagaku K.K.)
- Ad.5: ethylene-glycidyl methacrylate copolymer ("REXPEARL RA3150", made by Nippon Polyolefin K.K.)
- PGA: polyglycolic acid (made by Kureha Kagaku Kogyo K.K., specific gravity=1.6)
- Ny 11: nylon 11 ("RILSAN BESVOAFDA", made by Atochem Co.)

### [Examples 1-7, Comparative Examples 1-7]

As genuine working examples, a packaging product formed by enclosing a content material within a packaging material should by heat-treated. However, as the effect of boil or retort sterilization itself on the content material is well known to a skilled artisan, the opalescence characteristics of packaging film materials under boil or retort heat-treatment conditions were tested in the following manner.

Two film samples each measuring 5 cm × 5 cm was cut out from each of film-form plastic packaging materials having laminate structures shown in Table 1. (Note: In the following description including Tables, films having a laminate structure using "/" between layers are laminate films formed by co-extrusion, and films having a laminate structure using "|" between layers (of Example 4 and Comparative Example 4) are laminate films formed by dry lamination.). Each film sample of 5 cm × 5 cm was enclosed within a pouch of polyethylene-laminated aluminum sheet measuring 12 cm × 12 cm together with 10 ml of 25 wt.% sodium chloride aqueous solution (Examples 1-7) or tap water (Comparative Examples 1-6), and the pouch was hermetically sealed without leaving air therein. Two pouches prepared from each packaging material sample were immersed in hot water at 95°C and 120°C , respectively, for 0.5 hour, then cooled with tap water, and opened to take out the film samples. The film samples were then subjected to measurement of haze (according to JIS K6714). The measured results are shown in Table 1 together with haze values before the heat treatment. Incidentally, the heat treatment at 100°C was effected by immersion in hot water under the atmospheric pressure, and the heat treatment at 120°C was by immersion in hot water in a retort oven. Further, the films of Examples 2,3 and 5 were also subjected to the boil and/or retort heat treatment in 1 %-saline water, and the measured haze values are indicated in parentheses in parallel in the following table 1. The films of Examples 1, 2 and 5 were also subjected to the boil and/or retort heat treatment in 0.5%-saline water and the measured values are indicated in parallel in (( )) (double parentheses) in the following Table 1.

**Table 1**

| Example | Laminate structure | Total thickness | Haze (%)^{*} | | |
|---|---|---|---|---|---|
| | | | Before heat treatment | After heat treatment | |
| | ( thickness : in µm ) | (µm) | | 98°C × 0.5hr | 120°C × 0.5hr |
| 1 | EVOH/Ny6-66/Ad.1/LLDPE | 180 | 2 | 19 ((51)) | 66 ((74)) |
| | (43) (18) (18) (101) | | | | |
| Comp.1 | ditto | | 2 | 77 | 89 |
| 2 | Ny6/Ny6-66/Ad.1/LLDPE | 120 | 5 | 10 | 14 (13),((28)) |
| | (36) (10) (15) (59) | | | | |
| Comp.2 | ditto | | 5 | 11 | 71 |
| 3 | LLDPE/Ad.1/Ny6-66/Ad.1/VLDPE | 150 | 6 | 5 (8) | 7 (23) |
| | (36) (6) (43) (6) (59) | | | | |
| Comp.3 | ditto | | 6 | 14 | 61 |
| 4 | CPP/Ad.2/HB-Ny/CPP | 49 | 5 | 7 | 19 |
| | (15) (2) (15) (15) | | | | |
| Comp.4 | ditto | | 5 | 11 | 45 |
| 5 | EVA/Ad.3/EVOH/Ad.4/PP | 100 | 9 | 16 6 (13),((18)) | 13 |
| | (40) (3) (24) (3) (30) | | | | |
| Comp.5 | ditto | | 9 | 40 | 92 |
| 6 | PE/Ad.5/PGA/Ad.5/PE | 200 | 4 | 20 | 20 |
| | (60) (15) (50) (15) (60) | | | | |
| Comp.6 | ditto | | 4 | 82 | 93 |
| 7 | Ny6-66/Ad.1/LLDPE | 120 | 6 | 8 | 9 |
| | (46) (15) (59) | | | | |
| Comp.7 | ditto | | 6 | 18 | 79 |

| | | | | | |
|---|---|---|---|---|---|
| * The haze values indicated in parentheses for Examples 2, 3 and 5 were obtained in the case of immersion in 1 %-saline water; the haze values indicated in (( )) were obtained in the case of immersion in 0.5%-saline water; and the other haze values were obtained in the case of immersion in 25%-saline water. | | | | | |

### [Example 8 and Comparative Example 8]

Two film-form plastic packaging material samples each having a laminate structure shown in Table 2 below were subjected to the same treatments as in the above Examples 1-7 and Comparative Examples 1-7, including: cutting-out of film samples, enclosures within pouches, heat treatment at 98°C for 0.5 hour and 3.0 hours, and measurement of haze values before and after the heat treatment. The results are also shown in Table 2.

**Table 2**

| Example | Laminate structure | Total thickness (µm) | Haze (%) | | |
|---|---|---|---|---|---|
| | | | Before heat treatment | After heat treatment | |
| | ( thickness : in µm ) | | | 98°C × 0.5hr | 98°C × 3hrs |
| 8 | VLDPE/Ad.5/PGA/Ad.5/VLDPE | 200 | 3 | 15 | 32 |
| | (60) (15) (50) (15) (60) | | | | |
| Comp.8 | ditto | | 3 | 72 | 59 |

### [Example 9,10 and Comparative Example 9]

An EVOH-based laminate film ("PAIRFLEX SHEET FA-292N", made by Kureha Kagaku Kogyo K.K.; total thickness = 200 µm) having a laminate structure shown below was immersed in tap water and saturated sodium chloride (saline) water (having a concentration of ca. 27wt.%) and subjected to heat-treatments under conditions shown in Table 3. The states (particularly transparency) of the films after the immersion were evaluated by eye observation, and the results are also shown in Table 3.

| | Ny11/Ad.3/EVOH/Ny6-66/Ad.3/LLDPE/VLDPE | | | | | | |
|---|---|---|---|---|---|---|---|
| Thickness (µm) | (25) | (15) | (15) | (40) | (15) | (75) | (15) |

**Table 3**

| Example | Heating medium | Heating temp. (°C) | Heating time | | |
|---|---|---|---|---|---|
| | | | 1hour | 2hours | 4hours |
| Com.9 | tap water | 100 | opaque | opaque | opaque |
| 9 | sat. saline water | 105 | transparent | transparent | transparent |
| 10 | sat. saline water | 120 | transparent | a little turbid | a little turbid |

### [Reference Examples]

A polyglycolic acid-based laminate film having a laminate structure shown below was subjected to immersion in heating media shown in Table 4 at 100°C for indicated periods of time, and then taken out to be observed with eyes with respect to appearances, particularly with respect to transparency. The results are shown in Table 4.

| Ny11/Ad.3/EVOH/Ny6-66/Ad.3/LLDPE/VLDPE | | | | | |
|---|---|---|---|---|---|
| (60) | (15) | (50) | (15) | (15) | (60) |

### Evaluation by eye observation:

The evaluation results shown in Table 4 were obtained by evaluation with eyes of appearances and shown in Table 4 at 5 levels of opacity according to the following standard:
A: None. ( Haze ≦ 10%, A level of haze obtained after heat-treating the laminate film for 1 hour in a drying oven at 100 °C .)
B: Slight. (Haze=10-20%),
C: Moderate. (Haze=20-50%),
D: Much. (Haze=50-80%),
E: Extreme. (Haze ≧ 80%).

**Table 4**

| Heating medium ^{*} | Heating time | | |
|---|---|---|---|
| | 0.5 hr | 1.0 hr | 3.0 hrs |
| Pure warer | C | D | E |
| sat NaCl aq. (ca.27%) | A | A | A |
| 25% NaCl aq. | A | A | A |
| 15% NaCl aq. | A | A | A |
| 5% NaCl aq. | B | B | C |
| sat MgCl₂ aq. (ca.35%) | A | A | A |
| 10% MgCl₂ aq. | B | B | C |
| sat. CaCl₂ aq. (ca.37%) | A | A | A |
| 20% CaCl₂ aq. | A | A | A |
| 5% CaCl₂ aq_{.} | B | B | C |
| sat. KCl aq. (ca.22%) | A | A | A |
| 10% KCl aq. | B | B | C |
| sat Na₂SO₄ aq. (ca.33%) | A | A | A |
| 15% Na₂SO₄ aq. | B | C | D |
| 100% ethylene glycol aq. | A | A | A |
| 50% ethylene glycol aq. | A | A | B |
| 10% ethylene glycol aq. | B | B | D |

| | | | |
|---|---|---|---|
| *The following abbreviations are used. sat. : saturated aq. : aqueous solution | | | |

### [INDUSTRIAL APPLICABILITY]

As is understood from the above-described Examples, Comparative Examples and Reference examples, according to the heat-treating method for a packaging product of the present invention, the conventional boil hot water treatment or retort hot water treatment is subjected to a simple modification of causing the hot water to contain a water-soluble compound, whereby it becomes possible to remarkably suppress the opalescence of a packaging material causing disadvantage in appearance and transparency and also a lowering in gas-barrier property, encountered in the conventional boil hot water treatment or retort hot water treatment of a packaging product with a packaging material including a hydrophilic resin layer.

## Claims

1. A heat-treating method for a packaged product, comprising:
providing a packaged product formed by enclosing a content material within a packaging material comprising at least a layer of hydrophilic resin, and
heat-treating the packaged product with hot water at a temperature of at least 60°C for a period of 1 min. to ca. 3 hours, wherein the hydrophilic resin is a resin selected from the group consisting of polyamides, polyesters, polyorganic acids including polyamino acids, vinyl alcohol (co-)polymers and acid-modified olefin (co-)polymers that when formed as a 50 µ m-thick single-layered film and immersed in still hot water at 100° C for 30 min., the film is hydrolyzed or exhibits a haze value of 20% or higher so that the packaging material in the packaged product has a property of causing opalescence when the packaged product is heat-treated with hot water containing no water-soluble compound, and
the hot water is caused to contain a water-soluble compound selected from an inorganic electrolyte and a water-soluble organic compound at a concentration exceeding 0.1 wt.
% and effective to suppress the opalescence of the packaging material in terms of a lowering in haze of at least 10%.

2. A heat--treating method according to claim 1, wherein the hot water has a temperature of 60-100°C to effect a boiling heat-treatment.

3. A heat-treating method according to claim 1, wherein the hot water has a temperature exceeding 100°C to effect a retort heat-treatment.

4. A heat-treating method according to any of claims 1 to 3, wherein the hot water contains the water-soluble compound at a concentration of at least 1 wt. %.

5. A heat-treating method according to any of claims 1 to 4, wherein the water-soluble compound is an inorganic electrolyte.

6. A heat-treating method according to claim 5, wherein the water-soluble compound is a water-soluble inorganic salt.

7. A heat-treating method according to claim 6, wherein the water-soluble compound is a chloride selected from the group consisting of sodium chloride, magnesium chloride, and potassium chloride.

8. A heat-treating method according to claim 7, wherein the water-soluble compound is sodium chloride.

9. A heat-treating method according to any of claims 1 to 4, wherein the water-soluble compound is a water-soluble organic compound.

10. A heat-treating method according to claim 9, wherein the water-soluble compound is a water-soluble alcohol.

11. A heat-treating method according to any of claims 1 to 10, wherein the hydrophilic resin layer is a gas-barrier resin layer.

12. A heat-treating method according to claim 11, wherein the gas-barrier resin is selected from the group consisting of ethylene-vinyl alcohol copolymer, polyamide (co-)polymers, and aliphatic ester (co-)polymers.

13. A heat-treating method according to claim 12, wherein the gas-barrier resin is selected from the group consisting ethylene-vinyl alcohol copolymer, polymetaxylylene adipamide and glycolic acid (co-)polymer.

14. A heat-treating method according to any of claims 1 to 13, wherein the packaging material has a multi-layer structure.

15. A heat-treating method according to claim 14, wherein the hydrophilic resin layer is disposed as a surface layer contacting the hot water of the packaging material.

16. A heat-treating method according to claim 14, wherein the hydrophilic resin layer is disposed as an inner layer not directly contacting the hot water of the packaging material.

17. A heat-treating method according to claim 16, wherein the gas-barrier resin is glycolic acid (co-)polymer.

18. A heat-treating method according to any of the preceding claims, wherein the packaging material is heat-shrinked during the heat-treatment.

## Patentansprüche

1. Wärmebehandlungsverfahren für ein verpacktes Produkt, umfassend:
Bereitstellen eines verpackten Produktes, das durch Einschließen eines Inhaltsmaterials in einem Verpackungsmaterial, das wenigstens eine Schicht aus hydrophilem Harz umfasst, gebildet wird, und
Wärmebehandlung des verpackten Produktes mit heißem Wasser mit einer Temperatur von wenigstens 60°C für einen Zeitraum von 1 Minute bis ca. 3 Stunden, wobei das hydrophile Harz ein Harz ist, das aus der Gruppe, bestehend aus Polyamiden, Polyestern, organischen Polysäuren, einschließlich Polyaminosäuren, Vinylalkohol-(Co-)Polymeren und Säuremodifizierten Olefin-(Co-)Polymeren, ausgewählt ist, wobei, wenn das Harz als ein 50 µm-dicker einschichtiger Film geformt wird und in noch heißes Wasser mit 100°C für 30 Minuten eingetaucht wird, der Film hydrolysiert wird oder einen Trübungswert von 20% oder höher aufweist, so dass das Verpackungsmaterial in dem verpackten Produkt eine Eigenschaft hat, Opaleszenz zu verursachen, wenn das verpackte Produkt mit heißem Wasser, das keine wasserlösliche Verbindung enthält, wärmebehandelt wird, und
das heiße Wasser eine wasserlösliche Verbindung, die aus einem anorganischen Elektrolyten und einer wasserlöslichen organischen Verbindung ausgewählt ist, in einer Konzentration, die 0,1 Gew.-% übersteigt und wirksam ist, um die Opaleszenz des Verpackungsmaterials zu unterdrücken, ausgedrückt als Senkung der Trübung um wenigstens 10%, enthalten gelassen wird.

2. Wärmebehandlungsverfahren nach Anspruch 1, wobei das heiße Wasser eine Temperatur von 60 bis 100°C hat, um eine Wärmebehandlung unter Sieden durchzuführen.

3. Wärmebehandlungsverfahren nach Anspruch 1, wobei das heiße Wasser eine Temperatur hat, die 100°C übersteigt, um eine Retorten-Wärmebehandlung durchzuführen.

4. Wärmebehandlungsverfahren nach einem der Ansprüche 1 bis 3, wobei das heiße Wasser die wasserlösliche Verbindung in einer Konzentration von wenigstens 1 Gew.-% enthält.

5. Wärmebehandlungsverfahren nach einem der Ansprüche 1 bis 4, wobei die wasserlösliche Verbindung ein anorganischer Elektrolyt ist.

6. Wärmebehandlungsverfahren nach Anspruch 5, wobei die wasserlösliche Verbindung ein wasserlösliches anorganisches Salz ist.

7. Wärmebehandlungsverfahren nach Anspruch 6, wobei die wasserlösliche Verbindung ein Chlorid ist, das ausgewählt ist aus der Gruppe, bestehend aus Natriumchlorid, Magnesiumchlorid und Kaliumchlorid.

8. Wärmebehandlungsverfahren nach Anspruch 7, wobei die wasserlösliche Verbindung Natriumchlorid ist.

9. Wärmebehandlungsverfahren nach einem der Ansprüche 1 bis 4, wobei die wasserlösliche Verbindung eine wasserlösliche organische Verbindung ist.

10. Wärmebehandlungsverfahren nach Anspruch 9, wobei die wasserlösliche Verbindung ein wasserlöslicher Alkohol ist.

11. Wärmebehandlungsverfahren nach einem der Ansprüche 1 bis 10, wobei die hydrophile Harzschicht eine Gasbarriereharzschicht ist.

12. Wärmebehandlungsverfahren nach Anspruch 11, wobei das Gasbarriereharz ausgewählt ist aus der Gruppe, bestehend aus Ethylen-Vinylalkohol-Copolymer, Polyamid-(Co-)Polymeren und aliphatischen Ester-(Co-)Polymeren.

13. Wärmebehandlungsverfahren nach Anspruch 12, wobei das Gasbarriereharz ausgewählt ist aus der Gruppe, bestehend aus Ethylen-Vinylalkohol-Copolymer, Polymetaxylylenadipamid und Glycolsäure-(Co-)Polymer

14. Wärmebehandlungsverfahren nach einem der Ansprüche 1 bis 13, wobei das Verpackungsmaterial eine Mehrschichtenstruktur hat.

15. Wärmebehandlungsverfahren nach Anspruch 14, wobei die hydrophile Harzschicht als eine Oberflächenschicht, die mit dem heißen Wasser in Kontakt kommt, des Verpackungsmaterials angeordnet ist.

16. Wärmebehandlungsverfahren nach Anspruch 14, wobei die hydrophile Harzschicht als eine innere Schicht, die nicht direkt mit dem heißen Wasser in Kontakt kommt, des Verpackungsmaterials angeordnet ist.

17. Wärmebehandlungsverfahren nach Anspruch 16, wobei das Gasbarriereharz Glycolsäure-(Co-)Polymer ist.

18. Wärmebehandlungsverfahren nach einem der vorangehenden Ansprüche, wobei das Verpackungsmaterial während der Wärmebehandlung aufgeschrumpft wird.

## Revendications

1. Méthode de traitement thermique pour un produit emballé, comprenant :
· la fourniture d'un produit emballé formé en intégrant un contenu dans un matériau d'emballage comprenant au moins une couche de résine hydrophile, et
· le traitement thermique du produit emballé avec de l'eau chaude à une température d'au moins 60°C pendant 1 minute à environ 3 heures, dans laquelle la résine hydrophile est une résine choisie dans le groupe constitué de polyamides, polyesters, acides polyorganiques incluant des acides polyamino, des (co)polymères d'alcool vinylique et des (co)polymères d'oléfine modifiés par acide qui, lorsqu'ils sont formés en un film monocouche de 50 µm d'épaisseur et immergés dans de l'eau toujours chaude à 100°C pendant 30 minutes, le film est hydrolysé ou présente une valeur de turbidité de 20% ou plus, de sorte que le matériau d'emballage dans le produit emballé a une propriété consistant à provoquer l'opalescence quand le produit emballé est traité thermiquement avec de l'eau chaude ne contenant aucun composé soluble dans l'eau, et
· un composé soluble dans l'eau, choisi parmi un électrolyte inorganique et un composé organique soluble dans l'eau, est ajouté à l'eau chaude, à une concentration dépassant 0,1% en poids et efficace pour supprimer l'opalescence du matériau d'emballage en termes de réduction de la turbidité d'au moins 10%.

2. Méthode de traitement thermique selon la revendication 1, dans laquelle l'eau chaude a une température de 60-100°C pour effectuer un traitement thermique par ébullition.

3. Méthode de traitement thermique selon la revendication 1, dans laquelle l'eau chaude a une température qui dépasse 100°C pour effectuer un traitement thermique en autoclave.

4. Méthode de traitement thermique selon l'une quelconque des revendications 1 à 3, dans laquelle l'eau chaude contient le composé soluble dans l'eau à une concentration d'au moins 1% en poids.

5. Méthode de traitement thermique selon l'une quelconque des revendications 1 à 4, dans laquelle le composé soluble dans l'eau est un électrolyte inorganique.

6. Méthode de traitement thermique selon la revendication 5, dans laquelle le composé soluble dans l'eau est un sel inorganique soluble dans l'eau.

7. Méthode de traitement thermique selon la revendication 6, dans laquelle le composé soluble dans l'eau est un chlorure choisi dans le groupe constitué de chlorure de sodium, de chlorure de magnésium, et de chlorure de potassium.

8. Méthode de traitement thermique selon la revendication 7, dans laquelle le composé soluble dans l'eau est du chlorure de sodium.

9. Méthode de traitement thermique selon l'une quelconque des revendications 1 à 4, dans laquelle le composé soluble dans l'eau est un composé organique soluble dans l'eau.

10. Méthode de traitement thermique selon la revendication 9, dans laquelle le composé soluble dans l'eau est un alcool soluble dans l'eau.

11. Méthode de traitement thermique selon l'une quelconque des revendications 1 à 10, dans laquelle la couche de résine hydrophile est une couche de résine imperméable au gaz.

12. Méthode de traitement thermique selon la revendication 11, dans laquelle la résine imperméable au gaz est choisie dans le groupe constitué de copolymère d'éthylène-alcool vinylique, (co-)polymères de polyamide, et (co-) polymères d'ester aliphatique.

13. Méthode de traitement thermique selon la revendication 12, dans laquelle la résine imperméable au gaz est choisie dans le groupe constitué de copolymère d'éthylène-alcool vinylique, adipamide de polymétaxylylène et (co-) polymère d'acide glycolique.

14. Méthode de traitement thermique selon l'une quelconque des revendications 1 à 13, dans laquelle le matériau d'emballage a une structure multicouche.

15. Méthode de traitement thermique selon la revendication 14, dans laquelle la couche de résine hydrophile est disposée comme une couche de surface en contact avec l'eau chaude du matériau d'emballage.

16. Méthode de traitement thermique selon la revendication 14, dans laquelle la couche de résine hydrophile est disposée à une couche intérieure non directement au contact de l'eau chaude du matériau d'emballage.

17. Méthode de traitement thermique selon la revendication 16, dans laquelle la résine imperméable au gaz est un (co-)polymère d'acide glycolique.

18. Méthode de traitement thermique selon l'une quelconque des revendications précédentes, dans laquelle le matériau d'emballage est thermo-rétracté pendant le traitement thermique.
